# EUROPÄISCHE PATENTSCHRIFT

(11) **EP 1 912 922 B1**
(45) Veröffentlichungstag und Bekanntmachung des Hinweises auf die Patenterteilung: **21.01.2009**
(21) Anmeldenummer: 06776078.5
(22) Anmeldetag: 26.06.2006
(51) Int. Cl.: C07C 1/04, C10G 2/00

(54) **VERFAHREN ZUR ERZEUGUNG VON FLÜSSIGEN KOHLENWASSERSTOFFEN AUS GAS**
METHOD FOR PRODUCING LIQUID HYDROCARBONS FROM GAS
PROCEDE DE PRODUCTION D'HYDROCARBURES LIQUIDES A PARTIR DE GAZ

(30) Priorität: 28.07.2005 DE 102005035988
(43) Veröffentlichungstag der Anmeldung: 23.04.2008
(73) Patentinhaber: Wolf, Bodo Max, 09638 Lichtenberg (DE)
(72) Erfinder: Wolf, Bodo Max, 09638 Lichtenberg (DE)
(74) Vertreter: Patentanwälte Zellentin & Partner
(86) Internationale Anmeldenummer: PCT/EP2006/006135
(87) Internationale Veröffentlichungsnummer: WO 2007/012369

(56) Entgegenhaltungen:
- WO-A-99/21940

## Beschreibung

Die Erfindung betrifft ein Verfahren zur Erzeugung von flüssigen synthetischen Kohlenwasserstoffen aus einem Gas mit einem hohen Anteil an Wasserstoff und Kohlenmonoxid im Molekularverhältnis 1,5 bis 2,5 zu 1 nach dem Prinzip der Fischer-Tropsch-Synthese.

Das Anwendungsgebiet der Erfindung ist die Herstellung von flüssigen Brenn- und Kraftstoffen aus Synthesegas, das aus Erdgas oder flüssigen und festen Brennstoffen, insbesondere Biomasse wie Holz, Stroh, Energiepflanzen und/oder organischen Abfällen und Reststoffen durch partielle autotherme oder allotherme Oxidation mit nachfolgender Gasreinigung und -aufbereitung, z.B. CO-Konvertierung und CO₂-Wäsche, weiterhin als Synthesegaserzeugung bezeichnet, hergestellt wurde.

### Die Herstellung von flüssigen Kohlenwasserstoffen, insbesondere in Form von

Benzin, Dieselöl und Wachs aus Synthesegasen mit hohem Wasserstoff- und Kohlenmonoxidanteil ist seit 80 Jahren unter dem Begriff "Fischer-Tropsch-Synthese" bekannt und damit Stand der Technik. Die ersten Industrieanlagen arbeiteten in Deutschland mit Synthesegas, das aus Steinkohlenkoks durch Vergasung nach dem Wassergasverfahren hergestellt wurde. Die Anforderungen an die Gaserzeugung, methanarmes Gas mit großer Leistung, führten neben der Entwicklung der Festbettdruckvergasung auch zur Entwicklung der Winklervergasung, die weiter entwickelt, heute allgemein als Wirbelschichtvergasung noch Anwendung findet.

Die Notwendigkeit schwere Heizöle zu verwenden führte in der zweiten Hälfte des vorigen Jahrhunderts zur Entwicklung der Flugstromvergasung, also der partiellen Oxidation in einer Flammenreaktion, erst unter Normaldruck, später vorrangig unter Drücken von 2.5 bis 6 MPa (25 bis-60-bar) Die industrielle Anwendung der Kombination Kohlevergasung/Kraftstoffsynthese wurde vor allem in Südafrika vorangetrieben. In den letzten 20 Jahren beschäftigen sich aber auch mehrere Mineralölgesellschaften verstärkt mit der Entwicklung und industriellen Anwendung der Kombination Vergasung von Erdoder Erdölbegleitgas/Kraftstoffsynthese. Der Stand der Technik wird hier von Shell mit der Anlage in Bintulu, Malaysia, bestimmt.

Die mittelfristige Endlichkeit der fossilen Brennstoffe, insbesondere von Erdöl und Erdgas, war für das deutsche Unternehmen CHOREN Industries GmbH Anlass, 1995 die Entwicklung der Erzeugung von Synthesegas aus Biomasse zu beginnen und die Herstellung von erneuerbaren synthetischen Brenn- und Kraftstoffen aus diesem Gas nach der Fischer-Tropsch-Synthese mit marktüblichen Katalysatoren zu erproben. Es zeigte sich, dass die Kombination Synthesegaserzeugung aus Biomasse/Fischer-Tropsch-Synthese nach dem Vorbild des Standes der Technik nicht zuverlässig betrieben werden konnte. Die Reisezeit der Synthesekatalysatoren betrug nur mehrere Stunden bis wenige Tage. Danach waren die Katalysatoren zerfallen, so dass sich unbrauchbare Betriebszustände einstellten. Die Auswertung der elektronischen Aufzeichnungen schloss in den meisten Fällen Fehlbedienungen aus, so dass geschlussfolgert werden musste, dass die Ursachen in der Verfahrenstechnik der Prozesskombination Gaserzeugung, -reinigung, -aufbereitung, also der Synthesegaserzeugung und Synthese zu suchen sind. Geschlussfolgert wurde, dass der Zerfall des Katalysators bereits bei kleinen und kurzzeitigen Änderungen der Parameter, unter denen die Synthese abläuft, zur Flashverdampfung von Produkt in den Poren des Katalysators führen kann, was die Katalysatoren zerstört.

WO 99/21940 offenbart ein verfahren zur Erzengung von Brenn-,Synthese-und Reduktionsgas aus nachwachsenden und fossilen Brennstoffen, anderen Biomassen, müll oder Schlämmen.

Ziel der Erfindung ist die Verlängerung der Betriebszyklen der Synthese und die Vermeidung von Katalysatorschäden durch Zerfall der Katalysatorkörper.

Die technische Aufgabe besteht somit dann, technisch bedingte, insbesondere kurzzeitige Schwankungen der Partialdrücke der Gaskomponenten und damit der Temperaturen sowie des Druckniveaus, unter denen der Syntheseprozess abläuft, zu vermeiden.

Erfindungsgemäß wird die technische Aufgabe gelöst, indem zwischen den Prozessstufen Synthesegaserzeugung und Synthese ein erster Gasspeicher, der über dem Prozessdruck der Synthese arbeitet und zwischen der der Synthese nachgeschalteten Kondensation der flüssigen Produkte und der Purgegasabführung ein unter Prozessdruck arbeitender zweiter Gasspeicher eingebunden werden, die über einen Kreislaufkompressor und eine Druckreduzierung miteinander so verbunden sind, dass bei Druckabfall in der Zuführung von frischem Synthesegas aus der Synthesegaserzeugung zum ersten Gasspeicher der Druck des Gases in der Synthese, bei geöffneter oder geschlossener Purgegasabführung aus dem zweiten Gasspeicher konstant bleibt oder nur in dem Maße sinkt, wie durch den Stoffumsatz in der Synthese verursacht.

Die technische Realisierung der Erfindung ermöglicht unabhängig vom Betriebszustand der Synthesegaserzeugung einen Langzeitbetrieb der Synthese bis zur natürlichen Erschöpfung des Katalysators und das Wiederanfahren der Synthese nach Ausfällen der Synthesegaserzeugung mit voller Leistung. Der wirtschaftliche Vorteil der Erfindung liegt damit in der Bereitstellung eines Verfahrens zur Erzeugung von synthetischen Kraftstoffen, insbesondere von erneuerbaren synthetischen Kraftstoffen aus Biomasse mit hoher Zeitverfügbarkeit und damit Wirtschaftlichkeit sowie in der Vermeidung überhöhter Katalysatorkosten.

### Ausführungsbeispiel

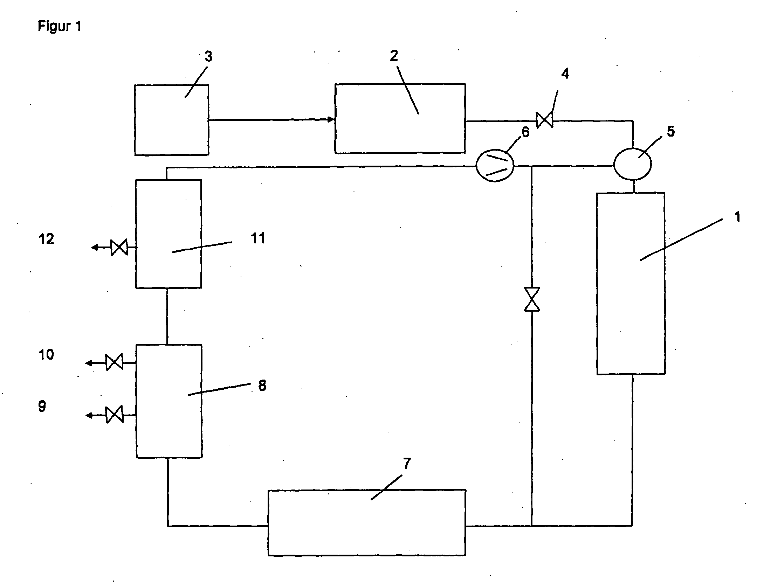

Die Erfindung wird mit nachfolgendem Beispiel, in dem die Synthese 1 bei einem Prozessdruck von 2.5Mpa (25 bar) arbeitet, und mit Hilfe von Figur 1 beschrieben. Das für die Synthese 1 erforderliche Gas wird durch partielle Oxidation von Biomasse, mit nachfolgender Gasreinigung und -aufbereitung sowie Verdichtung auf das Druckniveau des erfindungsgemäß ersten Gasspeichers 2, der im Beispiel bei einem Betriebsdruck von 3MPa (30 bar) arbeitet, durch die Synthesegaserzeugungsanlage 3 bereitgestellt und dem ersten Gasspeicher 2 zugeführt. Das im erfindungsgemäß ersten Gasspeicher 2 bei erhöhtem Druck bevorratete Synthesegas aus der Synthesegaserzeugung 3 wird über eine Gasdruckreduzierung 4 unter Zumischung 5 von durch Kompression 6 rückverdichtetem Gas aus dem Syntheseprozess selbst mit 2.5 mpa (25 bar) der Synthese 1 zugeführt. In der Synthese 1 setzt sich das Gasgemisch aus Synthesegas und prozessinternem Kreislaufgas an einem Katalysator teilweise zu Kohlenwasserstoffdämpfen und Wasserdampf um. Durch direkte Kühlung 7 des Gas-Dämpfe-Gemisches, z.B. durch Einspritzung von Wasser, werden die Dämpfe kondensiert, wodurch eine Separation 8 der Kondensate, bestehend aus Kohlenwasserstoffen und Wasser, vom Gas einerseits und von Wasser und flüssigen Kohlenwasserstoffen andererseits möglich wird, was die getrennte Ausschleusung von Wasser 9 und Kohlenwasserstoffen 10 ermöglicht. Das nach der Separation 8 von Gas und Kondensat vorliegende Gas wird dem erfindungsgemäß unter Prozessdruck der Synthese 1 arbeitenden zweiten Gasspeicher 11 zugeführt, der die Kompression 6 speist und aus dem das in der Synthese 1 nicht umsetzbare Purgegas 12 aus dem Verfahren abgeführt wird.

## Patentansprüche

1. Verfahren zur Erzeugung flüssiger Kohlenwasserstoffe aus Gas, das durch partielle Oxidation von Erdgas oder flüssigen und festen organischen Brennstoffen, insbesondere Biomasse wie Holz, Stroh, Energiepflanzen und/oder organischen Abfällen und Reststoffen durch partielle autotherme oder allotherme Oxidation mit nachfolgender Gasreinigung und -aufbereitung, z.B. CO-Konvertierung und C0₂-Wäsche in einer Synthesegaserzeugungsanlage hergestellt wurde und danach an Katalysatoren zu flüssigen Kohlenwasserstoffen umgewandelt wird, **dadurch gekennzeichnet, dass** zwischen den Prozessstufen Synthesegaserzeugung (3) und Synthese (1) ein erster Gasspeicher (2), der über dem Prozessdruck der Synthese arbeitet, und zwischen der der Synthese (1) nachgeschalteten Kondensation (7) der flüssigen Produkte und der Purgegasabführung (12) ein unter dem Prozessdruck der Synthese (1) arbeitender zweiter Gasspeicher (11) eingebunden werden, die über einen Kreislaufkompressor (6) und eine Druckreduzierung (4) so mit einander verbunden sind, dass bei Druckabfall in der Zuführung von frischem Synthesegas aus der Synthesegaserzeugung (3) zum ersten Gasspeicher (2) der Druck des Gases in der Synthese (1), bei geöffneter oder geschlossener Purgegasabführung (12) aus dem zweiten Gasspeicher (11), konstant bleibt oder nur in dem Maße sinkt, wie durch den Stoffumsatz in der Synthese verursacht.

## Claims

1. Method for producing liquid hydrocarbons from gas that was produced by partial oxidation of natural gas or from liquid and solid organic fuels, in particular biomass such as wood, straw, fuel plants and/or organic waste and recyclings by partial autothermic or allothermic oxidation followed by gas cleaning and processing, e.g. CO conversion and CO2 scrubbing, in a synthesis gas production facility and is then converted to liquid hydrocarbons on catalysts, **characterized in that** a first gas reservoir (2) operating above the process pressure of the synthesis is included between the process stages of synthesis gas production (3) and synthesis (1), and a second-gas reservoir (11) operating below the process pressure of the synthesis (1) is included between the condensation (7) of the liquid products downstream of the synthesis (1) and the purge gas discharge (12), said gas reservoirs being connected to each other by means of a closed circuit compressor (6) and a pressure reducer (4) such that, upon a drop of pressure in the feed of fresh synthesis gas from the synthesis gas production (3) to the first gas reservoir (2), the pressure of the gas in the synthesis (1) remains constant or drops only to the extent caused by the conversion of substances in the synthesis, while the purge gas discharge (12) from the second gas reservoir (11) is open or closed.

## Revendications

1. Procédé de production d'hydrocarbures liquides à partir de gaz produit par oxydation partielle de gaz naturel ou de combustibles organiques solides et liquides, en particulier la biomasse comme le bois, la paille, les plantes énergétiques et/ou les déchets et résidus organiques via oxydation partielle autotherme ou allotherme avec épuration et traitement des gaz subséquents, p. ex. conversion du CO et lavage du CO₂ dans une installation de production de gaz de synthèse et converti ensuite en hydrocarbures liquides sur catalyseurs, **caractérisé en ce que**, entre les étapes de procédé de production de gaz de synthèse (3) et de synthèse (1), un premier réservoir de gaz (2), qui fonctionne au-dessus de la pression de procédé de la synthèse, et, entre la condensation (7) des produits liquides en aval de la synthèse (1) et l'extraction de gaz de purge (12), un second réservoir de gaz (11), qui fonctionne en-dessous de la pression de procédé de la synthèse (1), sont intégrés, qui sont reliés de manière telle entre eux par un compresseur à circulation (6) et une réduction de pression (4) que, lors d'une baisse de pression de l'alimentation en gaz de synthèse frais issu de la production de gaz de synthèse (3) au premier réservoir de gaz (2), la pression du gaz dans la synthèse (1), l'extraction de gaz de purge (12) du second réservoir de gaz (11) étant ouverte ou fermée, reste constante ou ne diminue que dans la mesure causée par la conversion de matière dans la synthèse.
